# EUROPÄISCHE PATENTANMELDUNG

(11) **EP 0 889 119 A2**
(43) Veröffentlichungstag der Anmeldung: **07.01.1999**
(21) Anmeldenummer: 98111945.6
(22) Anmeldetag: 27.06.1998
(51) Int. Cl.: C12M 1/02, B01F 3/08

(54) **Verfahren und Vorrichtung zum Eintrag von Reagenzien in Reaktoren**

(30) Priorität: 30.06.1997 DE 19727731
(71) Anmelder: FORSCHUNGSZENTRUM JÜLICH GMBH, D-52425 Jülich (DE)
(72) Erfinder: Schmitz, Günter, 52428 Jülich (DE); Wandrey, Christan, Prof., 52428 Jülich (DE); Weuster-Botz, Dirk, Dr., 52070 Aachen (DE)

(57) **Zusammenfassung**

Die Erfindung betrifft ein Verfahren und einen Reaktor (1) mit denen insbesondere von Mikroorganismen durchgeführte, biochemische Reaktionen durchgeführt werden können. Nach dem Stand der Technik können einem Bioreaktor keine aggressiven Reagenzien, wie H₂O₂ zugeführt werden, ohne daß die Mikroorganismen beeinträchtigt oder gar getötet werden. Erfindungsgemäß erfolgt der Eintrag von aggressiven Reagenzien in den Reaktor (1) durch einen Rührer (2), welcher an den Spitzen der Rührblätter (13) Austrittsenden (6) für das aggressive Reagenz aufweisen, welches durch die Hohlwelle (12) des Rührers (2) gefördert wird. In einer anderen Ausführungsform wird das aggressive Reagenz durch einen porösen Festkörper (9) eingetragen, welcher in einen Bypass (7) des Reaktors (1) integriert ist.

## Beschreibung

Die Erfindung bezieht sich auf ein Verfahren zum Eintrag von Reagentien in Reaktoren nach Anspruch 1, insbesondere ein Verfrahren zum Eintrag von Oxidationsmitteln, wie Wasserstoffperoxid, und eine für die Durchführung des Verfahrens geeignete Vorrichtung.

Bei der Biotransformation werden Substrate mittels Mikroorganismen in einem Reaktor umgesetzt, dem zur Reaktion eine weitere Reaktionskomponente zugeführt wird. Ein besonderer Fall ist die Oxidation eines Substrates durch eine Biotransformation bei der aggressive Oxidationsmittel wie Wasserstoffperoxid (H₂O₂) oder Nitrat (NO₃) eingesetzt werden. Diese Stoffe greifen jedoch die zur Biotransformation benötigten Zellen bzw. Mikroorganismen an und wirken ab einer Stoff- und zellspezifischen Konzentration zelltoxisch. Zelltoxizität kann auch bei anderen Reagenzien auftreten, die keine Oxidationsmittel sind, jedoch auf eine andere Weise zellschädigend wirken, wenn sie in einer zu hohen Konzentration mit den Zellen in Kontakt gebracht werden. Handelt es sich bei der biochemischen Reaktion um eine Oxidation eines Substrates mit einem Oxidationsmittel, so kann als Oxidationsmittel direkt Sauerstoff oder zunächst H₂O₂ zugesetzt werden, welches erst bei einer chemischen oder biochemischen Reaktion in Sauerstoff (O₂) umgesetzt wird.

Erfolgt der Eintrag des Oxidationsmittels in die Bioreaktoren in Form von Sauerstoff, so finden nach dem Stand der Technik verschiedene Eintragverfahren Verwendung. Der Sauerstoffeintrag erfolgt in der Regel über die Zufuhr einer sauerstoffhaltigen Gasphase, die in intensiven Kontakt mit dem Reaktionsmedium gebracht wird, um Sauerstoff aus der Gasphase in die sich im Reaktor befindende Flüssigkeitsphase einzutragen. Ein Verfahren besteht in der Erhöhung des den Stofftransport treibenden Konzentrationsgefälles an Sauerstoff zwischen der Gasphase und der Flüssigphase durch Verwendung von Gasen mit hohem Sauerstoffanteil, wie Reinsauerstoff, mit Sauerstoff angereicherter Luft oder dem Einsatz höherer Drücke ( 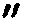 Turmbiologie") und/oder der Erhöhung der Phasengrenzfläche gas-flüssig über die der Sauerstoff transportiert werden muß mit Hilfe geeigneter Gasdispersionsorgane, wie Injektoren oder Düsen. Weiterhin ist die Verringerung des flüssigkeitsseitigen Stofftransportwiderstandes durch Erzeugung von Turbulenzen bekannt, der einer Erhöhung des Energieeintrages entspricht.

Ein wesentlicher, die erreichbare Mikroorganismenkonzentration und damit auch Produktionskonzentration und Produktivität limitierenden Faktor bei sauerstoffbenötigenden biotechnologischen Produktionsprozessen ist die begrenzte Sauerstoffeintragleistung der verwendeten Reaktoren. Bei Oxidationsreaktionen in Gegenwart organischer Lösungsmittel bei denen der benötigte Sauerstoff über die Gasphase eingetragen wird, kann es darüber hinaus zur Ausbildung explosionsartiger Gasgemische im Reaktor kommen. Daher wird in der Regel bei solchen Prozessen der Sauerstoffanteil im Gas auf Werte unterhalb der Explosionsgrenze reduziert (Zum Beispiel < 8 Vol.% bei Verwendung von n-Alkanen als organische Phase) und die Reaktoren explosionsgeschützt ausgeführt. Um dennoch den für die Oxidationsreaktion notwendigen Sauerstoff in ausreichender Menge in die wäßrige Flüssigphase einzutragen, müssen solche Reaktoren unter Überdruck betrieben werden, um trotz geringem Sauerstoffanteil in der Gasphase einen hohen Sauerstoffpartialdruck und damit den besseren Stofftransport des Sauerstoffs von der Gasphase in die Flüssigkeit zu erzielen.

In der Umweltbiotechnologie, zum Beispiel in der in-situ Sanierung von kontaminierten Böden wurden schon in Wasser gelöste Substanzen, wie H₂O₂ eingesetzt, die erst bei einer chemischen oder biochemischen Reaktion den benötigten Sauerstoff freisetzen. Hier wird mit H₂O₂ oder Nitrat versetztes Wasser in den Boden gepreßt, und erst die zur biologischen Sanierung im kontaminierten Boden befindlichen Mikroorganismen erzeugen durch die biochemische Reaktion vor Ort den für das Wachstum der Mikroorganismen benötigten Sauerstoff.

Auch zum Einsatz bei Fermentationsprozessen wurde die Verwendung von H₂O₂ zur in-situ Sauerstofferzeugung vorgeschlagen und im Labormaßstab getestet. Eine technische Anwendung erfolgte jedoch nicht, da keine schnellen homogene Verteilung des zugegebenen H₂O₂ möglich war. Bei einer lokalen Überdosierung können die Zellen sehr schnell durch das H₂O₂ oder andere Oxidationsmittel zerstört werden. Weiterhin treten bei der Sauerstoffversorgung über H₂O₂ aufgrund der fehlenden Gasphase sehr hohe CO₂-Konzentrationen in der wäßrigen Phase beziehungsweise im Lösungsmittel auf, die die Stoffwechselreaktionen des kultivierten Mikroorganismus stark verändern und zum Teil zum völligen Erliegen des Stoffwechsels führen.

Bei biochemischen Umsetzungen, die mit isolierten Enzymen durchgeführt werden, ist zum Schutz der reaktiven Enzyme sogar die Zugabe des isolierten Enzyms Katalase üblich, wenn bei der Reaktion das starke Oxidationsmittel H₂O₂ entsteht, um dieses schnell umzusetzen.

Es ist daher die Aufgabe der Erfindung ein Verfahren und eine Vorrichtung zu schaffen, mit denen chemische Umsetzungen mittels Mikroorganismen in Fermentern mittels agressiver Oxidationsmittel oder anderer zelltoxischer Reagentien durchgeführt werden können, ohne daß eine zelltoxische Wirkung eintritt.

Ausgehend vom Oberbegriff des Anpspruchs 1 wird die Aufgabe erfindungsgemäß gelöst mit dem im kennzeichnenden Teil des Anspruchs 1 angegebenen Merkmal.

Mit dem erfindungsgemäßen Verfahren und der Vorrichtung ist es nunmehr möglich, biochemische Umsetzungen von Substraten mittels Mikroorganismen mit zellschädigenden Reagentien durchzuführen ohne daß eine Zelltoxische Wirkung eintritt. Die Organismen können somit länger im Produktionsbetrieb genutzt werden, es werden Betriebsstillstände, bedingt durch den Austausch von Zellmaterial, vermieden.

Vorteilhafte Weiterbildungen der Erfindung sind in den Unteransprüchen angegeben.

Die Zeichnungen zeigen eine erfindungsgemäße Vorrichtungen in schematischer Form.

Es zeigt:
- Fig.1:: Einen Fermenter mit einem erfindungegemäß ausgestalteten Rührer.
- Fig.2:: Einen Fermenter, der einen Bypass mit Eintragssinterkörper aufweist.
- Fig.3:: Einen Fermenter mit Bypass zum Eintrag von Reagenz.
- Fig.4:: Eine Graphik mit einem Oxidationsverlauf von Inden zu Indenoxid mit induzierten Pseudomonas putida.
- Fig.5:: Eine Grafik mit der Abhängigkeit der Sauerstoffverbrauchsrate von der Prozeßzeit
- Fig. 6:: Den Querschnitt durch eine mikroporöse Kapillarmembran/Rohrmembran.

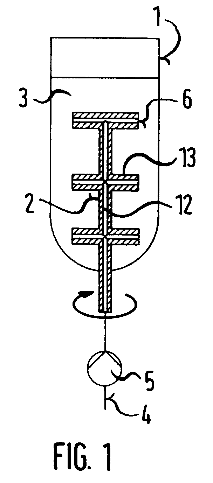

Figur 1 zeigt einen Reaktor 1, der mit einem Rührer 2 ausgestattet ist und der mit einer flüssigen Phase 3, welche mit den Mikroorgaminsmen und dem Substrat beschickt ist, beaufschlagt ist. Der Reaktor 1 steht mit einer Versorgungsleitung 4 für ein Reagenz in Verbindung, die mit einer Förderpumpe 5 ausgestattet ist und die in den Rührer 2 mündet. Die Versorgungsleitung 4 wird innerhalb des Rührers 2 in Form der Hohlwelle 12 fortgeführt und mündet in Austrittsenden 6, die sich auf den Rührblättern 13 befinden.

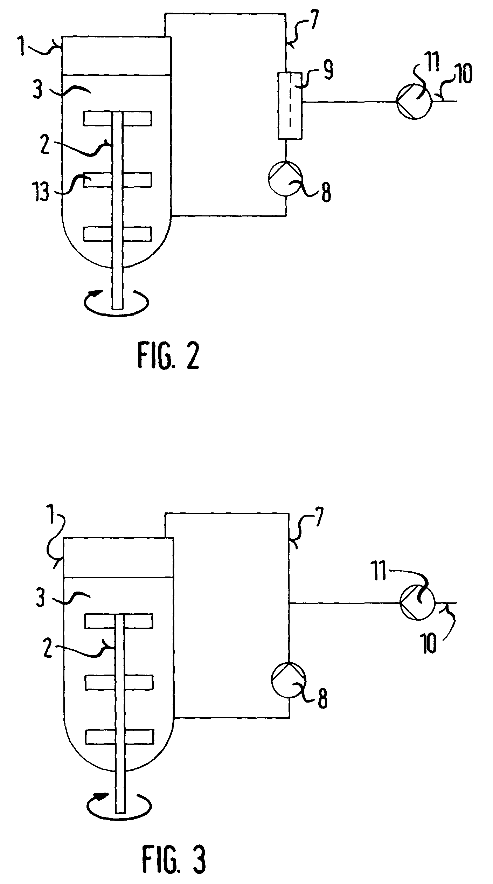

In Figur 2 sind den gleichen Vorrichtungsmerkmalen dieselben Bezugszeichen zugeordnet. Der Reaktor 1 trägt jedoch einen Bypass 7, der eine Pumpe 8 sowie eine mikroporöse Kapillarmembran/Rohrmembran 9 aufweist. In die mikroporöse Kapillarmembran 9 mündet eine Versorgungsleitung 10, mit einer Förderpumpe 11.

Figur 3 zeigt dieselbe Anordnung wie Figur 2 ohne die mikroporöse Kapillarmembran.

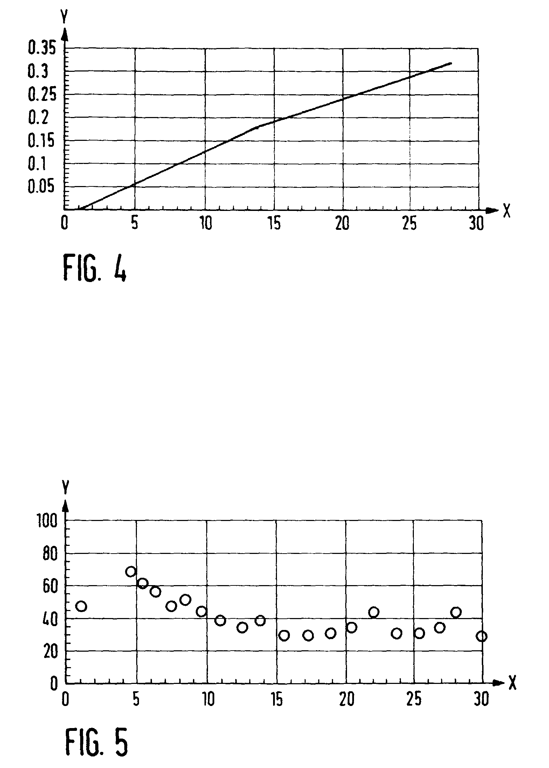

Figur 4 zeigt die Oxidation von Inden zu Indenoxid mit induzierten Pseudomonas putida Zellen. Es ist hierbei
- die Abzisse x:: Prozeßzeit in Minuten [min],
- die Ordinate y:: Konzentration an Indenoxid in Millimol [mM].

Figur 5 zeigt eine Grafik mit der Abhängigkeit der Sauerstoffverbrauchsrate von der Zeit, für die Oxidation von Inden zu Indenoxid mit induzierten Pseudomonas putida Zellen und H₂O₂ -Zugabe.

In ihr ist die
- Abzisse x:: Die Prozeßzeit [min]
- Ordinate y:: Die O₂-Verbrauchsrate in Millimol pro Liter und Stunde [mmol/(l×h)] .

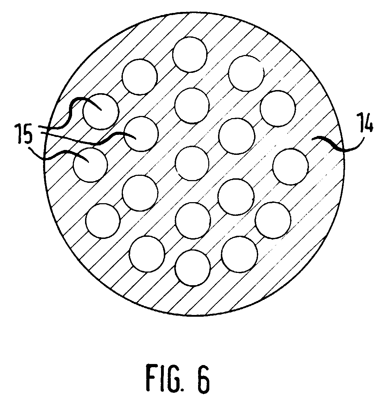

In Figur 6 ist der Querschnitt einer mikrporöse Kapillar- oder Rohrmembran 9 dargestellt. Sie besteht aus einer Keramik-Sintermasse 14, in die Kapillaren oder Kanäle 15 eingelassen sind.

Erfindungsgemäß wird das dem als Fermenter ausgebildetem Reaktor 1 zuzugebende Reagenz derart zugesetzt, daß im Zeitpunkt der Zugabe in die flüssige Phase 3 eine Verdünnung des Reagenzes erfolgt, so daß keine lokale Überdosierung des Reagenzes erfolgt, die zu einer zelltoxischen Wirkung führt.

Im folgenden soll das erfindungsgemäße Verfahren beispielhaft an der Zugabe von H₂O₂ in eine flüssige Phase 3 dargestellt werden.

Der in Figur 1 dargestellte Reaktor 1 wird mit einer Suspension an Mikroorganismen, die die flüsssige Phase 3 darstellt, beaufschlagt. In dieser Mikroorganismensuspension befindet sich das zu oxidierende Substrat.

Der Reaktor 1 wird nun in Betrieb genommen, indem der Rührer 2 in Rotation versetzt wird. Hierbei wird das zur Oxidation des Substrates benötigte H₂O₂ mittels der Förderpumpe 5 durch die Versorgungsleitung 4 in den Innenraum des Rührers 2 gepumpt. Dieser Innenraum wird durch eine Hohlwelle 12 gebildet, die sich in die Rührblätter 13 verzweigt. Er mündet in Austrittsenden 6 an den Rührblättern 13, die einen Austritt des H₂O₂ in die Suspension ermöglichen. Die Austrittsenden 6 befinden sich vorzugsweise an den Spitzen der Rührblätter 13, können aber auch auf der Oberfläche der Rührblätter 13 angeordnet sein und können mit einem feinen Bohrer hergestellt werden. Das H₂O₂ wird nun während des Rührens über den Rührer 2 in die Mikroorganismensuspension dosiert. Somit ist über die Kopplung der Dosierung mit der Rotation des Rührers 2 eine Verdünnung des H₂O₂ im Dosierungszeitpunkt gegeben, wodurch das Auftreten von lokalen Überdosierungen vermieden wird. Besonders wirkungsvoll ist die Verdünnung, wenn die Austrittsenden 6 sowohl auf den Oberflächen der Rührblätter 13, als auch auf den Spitzen der Rührblätter 13 verteilt sind. Es ist auch denkbar einige Austrittsenden 6 auf der Hohlwelle 12 anzusiedeln, jedoch handelt es sich hierbei um eine weniger bevorzugte Ausführungsform. Die Querschnitte der Austrittsenden 6 betragen idealerweise 0,1 bis 10 mm hängen jedoch von der Konzentration des H₂O₂, der Reaktor- oder Fermentergröße, das heißt, den zu versorgende Flüssigkeitsvolumen ab. Vorzugsweise wird der Rührer 2 derart ausgestaltet sein, daß mehrere Rührblätter 13 auf verschiedenen Ebenen der Rührerwelle angeordnet sind. Dies führt zu einer besonders guten Verteilung des in die Mikroorganismensuspension eizubringenden Reagenzes. Ein Rührer 2 mit Rührblättern 13 auf einer Ebene der Rührwelle führt jedoch ebenfalls zu guten Ergebnissen. Weiterhin können die Rührblätter 13 aus poröser Keramik oder porösen Metall- oder Glassinterkörper ausgebildet sein, durch die das Reagenz in die Suspension eingetragen werden kann. An Stelle der Hohlwelle 12 kann auch eine andere, flexible Zuleitung für das Reagenz treten.

In der Ausführungsform nach Figur 2 wird der mit der Mikroorganismensuspension beschickte Reaktor 1 mit einem Rührer 2 betrieben, der konventionell ausgestaltet ist, das heißt, der keine Austrittsenden 6 für H₂O₂ an den Rührblättern 13 aufweist. Bei dieser Ausführungsform erfolgt die Zugabe des H₂O₂ in den Reaktor 1 über einen Bypass 7. Der Querschnitt des Bypasses 7 hängt von der Größe des Reaktors 1 ab. Vorzugsweise weist der Bypass 7 einen großen Querschnitt auf und bildet eine kurze Durchlaufstrecke für die Suspension, so daß sich eine kurze Verweilzeit der Zellen im Umlauf einstellt. Hierbei wird das zu dosiernde H₂O₂ mittels der Versorgungsleitung 10 über die Förderpumpe 11 in die mikroporöse Kapillarmembran 9, welche von der mit Substrat beladenen Mikroorganismensuspension durchströmt wird, eingebracht. Bei der mikroporösen Kapillarmembran 9 bzw. der mikroporösen Rohrmenbran handelt es sich vorzugsweise um einen Patronenförmig ausgebildeten Festkörper, der in Fließrichtung der mit Mikroorganismen beladenen Suspension Kapillaren, bzw. Kanäle 15 aufweist, durch die die Suspension fließen kann. Die Summe der Querschnittsflächen der Kapillaren oder Kanäle 15 ist dabei vorzugsweise gleich dem Strömungsquerschnitt des Bypasses 9, so daß der Durchmesser der Patrone etwas größer ist, als der des Bypasses 9 und daß weiterhin kein Druckabfall beim Passieren der Patrone erfolgt. Das H₂O₂ wird somit mit dem Kreislauf des Bypasses 9 in den Reaktor 1 eingebracht, in dem eine zusätzliche Vermischung durch den Rührer 2 erfolgt. Die erfindungsgemäße Verdünnung tritt hierbei beim Austritt des H₂O₂ aus der mikroporösen Kapillarmembran 9 in den zirkulierenden Mikroorganismensuspensionkreislauf auf. Die mikroporöse Kapillarmembran 9 stellt eine sehr hohe Austrittsoberfrläche für das H₂O₂ zur Verfügung, was eine Verdünnung im Dosierungszeitunkt bewirkt. An der rauhen Oberfläche des mikroporösen Kapillarmembran 9 bilden sich Turbulenzen der zirkulierenden flüssigen Phase 3 aus, die zu einer schnellen Vermischung und somit Verdünnung des agressiven Oxidationsmittels führen. Somit können keine hohen lokalen H₂O₂ - Konzentrationen auftreten, die zu einer zellschädigenden Wirkung für die Mikroorganismen führen könnten. Durch das Rühren mittels des Rührers 2 wird diese Wirkung noch verstärtkt. Es ist auch denkbar, eine mikroporöse Kapillarmembran 9 in den Innenraum des Reaktors 1 zu integrieren und innenhalb des Reaktors 1 eine Strömung aufrecht zuerhalten, die die selbe Wirkung hat, wie der Bypass 7 der Figur 2. Selbstverständlich können in einer Vorrichtung eine mikroporöse Kapillarmembran 9 und ein mit Öffnungen ausgestatteter Rührer 2 gleichzeitig betrieben werden, jedoch wird eine von beiden Möglichkeiten in der Regel ausreichen, um die erfindungsgemäße Wirkung hervorzurufen. Als mikroporöse Kapillarmembran 9 kann im weiteren Sinne auch eine Rohrmembran sein. Die mikroporösen Membranen sind vorzugsweise aus Keramik und haben eine Porengröße, die geeignet ist, Mikroorganismen vor einem Eintritt in das poröse Material zu hindern. Die Größenordnung von Mikroorganismen liegt in der Regel bei ca. 0,2 µm, so daß die Porengröße ≤ 0,2 µm betragen kann. Aus der Siebtechnik ist bekannt, daß auch Siebgewebe mit einer etwas größeren Maschenweite als der eines Partikelquerschnittes dazu in der Lage sind diese Partikel zurückzuhalten, so daß die Porengröße die Grenze von 0,2 µm auch überschreiten kann. Die Porengröße richtet sich nach der Größe und Geometrie des Mikroorganismen. Als oberster Grenzwert für die Porengröße können 10 µm angegeben werden. Für die mikroporösen Membranen 9 können auch andere Materialien, wie Metallsinterkörper oder Glas zum Einsatz kommen.

In einer in Figur 3 dargestellten, vereinfachten Form der Figur 2 wird das H₂O₂ ohne eine mikroporöse Kapillarmembran 9 in den Bypass 7 eingebracht. Beim Beispiel von H₂O₂ als Oxidationsmittel können alle denkbaren Konzentrationsmengen eindosiert werden. Es hat sichn jedoch als besonders vorteilhaft herausgestellt, 0,5-5%ige H₂O₂ -Lösung zuzugeben, die erfindungsgemäß beim Eintritt in die flüssige Phase 3 verdünnt wird. Selbstverständlich können auch andre Oxidationsmittel, wie NO₃⁻ oder andere Reagenzien, die zelltoxisch wirken können, mit dem erfindungsgemäßen Verfahren und der Vorrichtung einer Mikroorganismensuspension zugegeben werden. Die Zugabe kann dabei kontinuierlich oder in Intervallen erfolgen. Die Zugabegeschwindigkeit und die Geschwindigkeit mit der gemäß der Vorrichtung in Figur 1 gerührt und gemäß der Vorrichtung in Figur 2 die flüssige Phase 3 in demn Bypass 9 zirkuliert, hängt von der zellschädigenden Wirkung des Reaktanden sowie der Reaktionskinetik ab. In Abhängigkeit von der Art der Organismen wird auch die mit dem erfindungsgemäßen Verfahren in der flüssigen Phase 3 einzustellen de stationäre Konzenatration an Reagenz unterschiedlich sein. Die zur Durchführung des Verfahrens idealerweise einzustellenden Parameter können jedoch durch Versuch ermittelt werden, wenn man die Empfindlichkeit des Mikroorganismus bezüglich verschiedener Reagenzien kennt.

Das erfindungsgemäße Verfahren kann mit allen für eine Biotranfsformation geeigneten Mikroorganismen betrieben werden. Als Biotransformation im Sinne der Erfindung sind alle biochemischen Reaktionen zu verstehen, bei denen ein Substrat mittels eines Mikroorganismus zu einem Produkt umgesetzt wird. Als Mikroorganismen können beispielsweise alle Pseudomonas- oder Bacillusstämme oder alle Mikroorganismen eingesetzt werden, die Katalase-aktiv sind. Beispiele für Substrate sind bei Oxidationsreaktionen: ungesättigte Kohlenwasserstoffe, Alkene, aromatische Alkene oder Alkine und deren funktiononalisierte Derivate.

In einer besonders vorteilhaften Ausgestaltung des erfindungsgemäßen Verfahrens wird in die flüssige Phase, welche aus Wasser und den Mikroorganismen mit dem Substrat besteht, eine zweite organische Komponente einemulgiert. Diese kann beispielsweise aus einem Lösungsmittel, wie Hexan, Toluol, Silikonöl, welches extrem untoxisch für Mikroorganismen ist, oder deren Gemischen bestehen. Somit kann sich ein Verteilungsgleichgewicht der Komponenten in den beiden emulgierten Phasen einstellen, welches das Verfahren begünstigt. Die Mikroorganismen verbleiben dabei überwiegend in der wässrigen Phase. Aufgrund des Lösungsgleichgewichtes ist das Substrat in der organischen Phase angereichert und die Konzentration an Substrat in der wässrigen Phase ist geringer. Dies hat den Vorteil daß möglicherweise vorhandene für Mikroorganismen zelltoxische Konzentrationen des Substrates in der wässrigen Phase nicht erreicht werden. Durch die Biotransformation wird das Substrat jedoch verbraucht, wodurch über das Lösungsgleichgewicht weiteres Substrat aus der organischen Phase in die wässrige Phase diffundiert. Die organische Phase wirkt somit als Vorrat, der immerwieder Substrat nachliefert. Je nach Löslichkeit des Substrates in der organischen Phase wird das Substrat mehr oder weniger in die wässerige Phase abgegeben. Somit kann durch wahl des Lösungsmittels auch die feindosierte Abgabe des Substrates in die wässrige Phase gesteuert werden. Dies ist insbesondere dann von Vorteil, wenn das Substrat seinerseits zellschädigende Wirkung aufseisen kann. Nach der Biotansformation wird das Reaktionsprodukt in die wässrige Phase ausgeschieden. Es steht seinerseits im Lösungsgleichgewicht mit der organischen Phase und kann von ihr durch Lösung oder Adsorption anderen Oberfläche aufgenommen und somit dem Reaktionsgleichgewicht entzogen werden. Somit wird ebenfalls zellschädigende Wirkung des Produktes auf den Mikroorganismus vermieden. Es wird ersichtlich, daß durdch die Wahl des organischen Lösungsmittels ein ideales Reaktionsgleichgewicht für die Biotransformation maßgeschneidert werden kann, das natürlich bei allen erfindungsgemäßen Vorrichtungen Anwendung finden kann. Die Biotransformation in 2-Phasensystemen, welche ein organisches Lösungsmittel beinhalten wurde bisher auf Grund von Explosionsgefahr gemieden. Mit dem erfindungsgemäßen Verfahren ist es erstmals möglich, den Vorteil der 2-phasigen Reaktionssysteme problemlos zu nutzen. Eine Oxidation von Substraten mittels eines von O₂ verschiedenen Oxidationsmittels war nämlich auf zellschonende Weise bisher nicht möglich. Explosionsgefahr kann somit trotz des Einsatzes organischer Lösungsmittel vermieden werden.

In einer besonders vorteilhaften Ausführungsform des erfindungsgemäßen Verfahrens wird der Eintrag von H₂O₂ mittels eines H₂O₂-Sensors derart reguliert, daß keine zelltoxische H₂O₂ -Konzentration erreicht wird. In vergleichbarer Weise kann die Zugabe jedes anderen Reagenzes in einer nicht-zelltoxischen Form erfolgen.

Wesentlicher Vorteil des erfindungsgemäßen Verfahrens, insbesondere zur Durchführung von Oxidationsreaktionen mit Mikroorganismen unter Wasserstoffperoxid-Zugabe zur *in-situ* Sauerstoffversorgung in einem Bioreaktor ist die weitaus höhere Sauerstoffeintragsleistung, da im Vergleich zu der Begasung mit O₂ kein Stofftransport über eine Pasengrenze mehr notwendig ist und somit höhere Zelldichten und höhere Produktivitäten ermöglicht werden können.

Weiterhin entsteht keine gefährliche sauerstoffhaltige Gasphase im Bioreaktor, da Wasserstoffperoxid entsprechend der benötigten Sauerstoffmenge nachdosiert wird und damit nicht soviel Sauerstoff bei der Umsetzung von Wasserstoffperoxid entsteht, daß sich eine Gasphase ausbilden könnte. Eine sauerstoffhaltige Gasphase kann jedoch bei Vorhandensein von brennbaren Lösungsmitteln zu einer explosionsgefährlichen Mischung führen.

Da kein Gaseintrag erforderlich ist, entsteht kaum Schaumbildung. Damit erhöht sich die Prozeßsicherheit. Die Anlagenkosten sind niedriger, da keine Kompressoren und Sterilfilteranlagen zur Sterilgasversorgung, keine Gasmischstation und kein Druckreaktor notwendig sind. Ein Austrag von während der Reaktion begildetem CO₂ oder anderen gasförmigen Reaktionsprodukten ist bekenkenlos möglich, da keine explosionsgefährlichen O₂/Lösungsmittel-Gemische mit ausgetragen werden können. Somit kann bei Biotransformationen eine Vergiftung der Mikroorganismen durch CO₂ vermieden werden.

### BEISPIEL

Oxidation von Inden mit *Pseudomonas putida* Zellen und Wasserstoffperoxid

Das aerobe Bodenbakterium *Pseudomonas putida* wird kontinuierlich in einem 2 1 Standardrührreaktor (Arbeitsvolumen 1,4 1) mit Essigsäure als Kohlenstoffquelie bei einer Verweilzeit von 3.5 Stunden kultiviert. Die Mediumszusammensetzung und Reaktionsbedingungen ist in der Tabelle angegeben. Die Begasung erfolgt mit Luft (1.5 1/min.), dem ein Stickstoffstrom (0.5 1/min.) mit gasförmigen Styrol (28 g Styrol/m³ Stickstoff)
beigemischt wird. Styrol dient zur Induktion der Mikroorganismen. Es wird im Fließgleichgewicht eine Zellkonzentration von 2.85 g Biotrockenmasse / 1 bei vollständigem Acetatverbrauch erreicht.

Die induzierten Zellen werden in den Rührreaktor 1 zur Biotransformation überführt. Hier erfolgt die Mischung mit denselben Anteilen (1:1:1) 50 mM Phosphatpuffer pH 7.0 sowie Cosubstratlösung (20 mM Phenylessigsäure in 50 mM Phosphatpuffer), sowie kultiviertes Bodenbakterium Pseudomonas putida, bevor bei einer Reaktionstemperatur von 30 °C Inden als 2. organische Phase zugegeben wird und eine Emulsion hergestellt wird. Die Cosubstratlösung ist erforderlich, um den bei der Biotransformation verbrauchten Cofaktor (NADH) zu regenerieren. Inden löst sich in der wäßrigen Phase bis zu einer Konzentration von 2 mM. Das in der wäßrigen Phase gelöste Substrat wird von den induzierten Zellen zu Indenoxid umgesetzt, sobald als Sauerstoffquelle Wasserstoffperoxid (4%ige wässrige Lösung) zugegeben wird. Die Zugabe der Wasserstoffperoxidiösung erfolgt dabei so, dass der mit einer Sauerstoffelektrode im Reaktor gemessene PO₂ auf Werte zwischen 10 - 30 % eingestellt wird. Die Bildung von Indenoxid ist über einen Zeitraum von 30 Minuten in Figur 5 dargestellt. Der Sauerstoffverbrauch in Figur 5 resultiert aus der Oxidationdes Inden zu Indenoxid ind dem Lebendverbrauch der Pseudomonas putida-Zellen. Die Zugabe an H₂O₂ -Lösung wird mit Hilfe einer oder merherer im Reaktor 1 angebrachten Sauerstoffkonzentrationsmesssonden und eines Regelkreises mit einer Dosiereinrichtung für H₂O₂ - Lösung geregelt, so daß nur soviel H₂O₂ -Lösung in den Reaktor gefördert wird, daß der bei der biochemischen Spaltung des Wasserstoffperoxides gebildete Sauerstoff vollständig von den Mikroorganismen zur Oxidation des organischen Substrats verwendet oder verstoffwechselt wird und damit keine Zunahme des Sauerstoffkonzentration im Reaktor 1 auf unphysiologische Konzentrationen oder sogar die Ausbildung einer sauerstoffhaltigen Gasphase erfolgt, beziehungsweise damit keine Abnahme der Sauerstoffkonzentration unter limitierende Werte auftritt. Zur Sicherstellung, daß bei dieser Biotransformation kein Sauerstoff über die Gasphase eingetragen wird, kann der Reaktor 1 mit Stickstoff überlagert werden. Bei Blindversuchen ohne Zellen kann nach Zugabe von Katalase keine Indenoxidbildung gemessen werden. Bei Blindversuchen ohne Wasserstoffperoxidzugabe sinkt der p0₂, auf Null; eine Indenoxidbildung konnte jedoch nicht festgestellt werden.

**Tabelle**

| Reaktionsbedingungen und Mediumszusammensetzung *Pseudomonas putida* | |
|---|---|
| Natriumacetat | 4,92 g/1 |
| Di-Kaliumhydrogenphosphat | 1,55 g/1 |
| Natriumdihydrogenphosphat | 0,85 g/1 |
| Ammoniumsulfat | 2,0 g/1 |
| Magnesiumchlorid *6 H₂0 | 0,1 g/1 |
| Kupfersulfat * 5 H₂0 | 0,2 mg/1 |
| Kobaltchlorid * 6 H₂0 | 0,4 mg/1 |
| Eisen(II)-sulfat * 7 H₂0 | 5,0 mg/1 |
| Natriummolybdat*2 H₂0 | 0,2 mg/1 |
| Zinksulfat * 7 H₂0 | 2,0 mg/1 |
| Mangan(II)-chlorid * 2 H₂0 | 1,0 mg/1 |
| EDTA | 10,0 mg/1 |
| pH | 7,0 |
| Titration mit NAOH | 2,0 mol/1 |
| T | 30 °C |
| Rührerdrehzahl | 1450 1/min |
| Zuluft | 2,0 1/min |

Mit dem erfindungsgemäßen Verfahren ist es nunmehr möglich, feinste Dosierungen an Reagenzien in Reaktoren vorzunehmen. Insbesondere können nicht-gasförmige Oxidationsmitel und andere flüssige Reagentien ohne lokale Überdosierung in Reaktoren oder Fermenter eingebracht werden. Die Folgen der Zelltoxizität eines Reagenzes werden im Fall des Beriebs eines Bioreaktors vollständig vermieden. Weiterhin wird eine eventuelle explosive Gasatmosphäre vermieden. Selbstverständlich können die erfindungsgemäße Vorrichtung und das Verfahren auch zur kinetisch kontrollierten Zugabe von Reagentien bei allen biochemischen und chemischen Reaktionen eingesetzt werden. Von dem Begriff Reagenzien sind erfindungsgemäß auch Stoffe umfaßt, die nicht chemisch reagieren, sondern lediglich untergemischt werden.

## Patentansprüche

1. Verfahren zum Eintrag von einem Reagenz in einen mit einem Medium (3) beaufschlagten Reaktor (1),
dadurch gekennzeichnet,
daß das Reagenz im Zeitpunkt des Eintrags des Reagenzes in den mit dem Medium (3) beaufschlagten Reaktor (1) an der Eintrittsstelle (6,9,20) eine sofortige Verdünnung erfährt.

2. Verfahren nach Anspruch 1,
dadurch gekennzeichnet,
daß das Reagenz nach dem Eintrag in das Medium (3) mit dem Medium (3) unmittelbar vermischt wird.

3. Verfahren nach Anspruch 1 oder 2,
dadurch gekennzeichnet,
daß das Medium (3) eine Flüssigkeit ist.

4. Verfahren nach einem der Ansprüche 1 bis 3,
dadurch gekennzeichnet,
daR das Reagenz ein nicht-gasförmiges Oxidationsmittel ist.

5. Verfahren nach einem der Ansprüche 1 bis 4,
dadurch gekennzeichnet,
daß das Reagenz über einen Rührer (2), der an seinen Rührblättern (13) Austrittsenden (6) aufweist, in das Medium (3) eingebracht wird.

6. Verfahren nach Anspruch 5,
dadurch gekennzeichnet,
daß das Reagenz an Austrittsenden (6), welche an den Spitzen der Rührblätter (13) positioniert sind in das Medium (3) eingetragen wird.

7. Verfahren nach Anspruch 5 oder 6,
dadurch gekennzeichnet,
daß das Reagenz durch einen Rührer (2) in das Medium (3) eingetragen wird, der mindestens zwei Ebenen von Rührblättern (13) aufweist, welche Austrittsenden (6) aufweisen.

8. Verfahren nach einem der Ansprüche 1 bis 4,
dadurch gekennzeichnet,
daß das Reagenz über einen die Austrittsoberfläche vergrößernden Festkörper in das Medium (3) eingetragen wird.

9. Verfahren nach Anspruch 8,
dadurch gekennzeichnet,
daß der Festkörper eine mikroporöse Kapillar- oder Rohrmembran (9) ist.

10. Verfahren nach Anspruch 8 oder 9,
dadurch gekennzeichnet,
daß das Medium (3) den die Austrittsoberfläche vergrößernden Festkörper durch eine Bypassführung (7) durchläuft, welche am Reaktor (1) angebracht ist.

11. Verfahren nach einem der Ansprüche 8 bis 10,
dadurch gekennzeichnet,
daß das Medium (3) im Reaktor (1) durchmischt wird.

12. Verfahren nach einem der Ansprüche 1 bis 12,
dadurch gekennzeichnet,
daß ein Substrat mittels eines Mikroorganismus mit einem Oxidationsmittel umgesetzt wird, welches eindosiert wird.

13. Verfahren nach Anspruch 12,
dadurch gekennzeichnet,
daßdas Oxidationsmittel H₂O₂ ist.

14. Verfahren nach Anspruch 13,
dadurch gekennzeichnet,
daß der Eintrag von H₂O₂ mittels einer Sauerstoffelektrode reguliert wird, die den über den H₂O₂ - Eintrag gebildeten Sauerstoffgehalt feststellt.

15. Verfahren nach Anspruch 14,
dadurch gekennzeichnet, daß
die Sauerstoffkonzentration derart geregelt wird, daß sich keine Sauerstoffhaltige Gasphase ausbildet.

16. Verfahren nach Anspruch 14 oder 15,
dadurch gekennzeichnet,
daß die Sauerstoffkonzentration derart geregelt wird, daß keine Abnahme der Sauerstoffkonzentration unter die Oxidation limitierenden Werte auftritt.

17. Verfahren nach einem der Anpsprüche 13 bis 16,
dadurch gekennzeichnet,
daß der Eintrag von H₂O₂ mittels eines H₂O₂ -Sensors derart geregelt wird, daß keine zelltoxische H₂O₂ - Konzentration erreicht wird.

18. Reaktor (1) mit Mitteln zum Eintrag eines Reagentien in ein Medium (3),
dadurch gekennzeichnet,
daß die Mittel (2,6,9) zum Eintrag von Reagentien in das Medium (3) im Eintragszeitpunkt eine sofortige Verdünnung des Reagenzes an der Eintrittsstelle (6,9) bewirken.

19. Reaktor (1) nach Anspruch 18,
dadurch gekennzeichnet,
daß das Mittel zum Eintrag von Reagentien in das Medium (3) ein Rührer (2) ist, dessen Rührblätter (13) Austrittsenden (6) für den Eintrag des Reagenzes in das Medium(3) aufweisen, die mit einer Hohlwelle (12) in Verbindung stehen, welche eine Versorgung mit dem Reagenz gewährleistet.

20. Reaktor (1) nach Anspruch 19,
dadurch gekennzeichnet,
daß die Austrittsenden (6) an den Spitzen des Rührblätter (13) positioniert sind.

21. Reaktor (1) nach Anspruch 18,
dadurch gekennzeichnet,
daß das Mittel zum Eintrag von Reagentien in das Medium (3) ein poröser Festkörper (9) ist.

22. Reaktor (1) nach Anspruch 21,
dadurch gekennzeichnet,
daß sich der poröse Festkörper (9) in einem Bypass(7) des Reaktors (1) befindet.

23. Reaktor (1) nach Anspruch 21 oder 22,
dadurch gekennzeichnet,
daß der poröse Festkörper (9) eine Kapillar- oder Rohrmembran ist.

24. Reaktor (1) nach Anspruch 23,
dadurch gekennzeichnet,
daß die Querschnittsfläche (15) für den Durchtritt des Mediums (3) durch den porösen Festkörper (9) gleich der Querschnittsfläche des Bypasses (7) ist.

25. Reaktor (1) nach einem der Ansprüche 21 bis 24,
dadurch gekennzeichnet,
daß der poröse Festkörper (9) aus Keramik ist.

26. Reaktor (1) nach einem der Ansprüche 18 bis 25,
dadurch gekennzeichnet,
daß er Mittel zur Regulierung der Eindosierung des Reagentien aufweist.

27. Reaktor (1) nach Anspruch 26,
dadurch gekennzeichnet,
daß das Mittel zur Regelung der Eindosierung des Reagenzes eine Sauerstoffregeleinrichtung ist.

28. Rührer (2),
dadurch gekennzeichnet,
daß er Rührblätter (13) mit Austrittsenden (6) aufweist, die mit Mitteln (12) zum Eintrag von einem Reagenz in Verbindung stehen.

29. Rührer (2) nach Anspruch 28,
dadurch gekennzeichnet,
daß das Mittel (12) zum Eintrag eines Reagenzes eine Hohlwelle ist.
